# EUROPEAN PATENT APPLICATION

(11) **EP 0 727 206 A2**
(43) Date of publication of application: **21.08.1996**
(21) Application number: 96200041.0
(22) Date of filing: 09.01.1996
(51) Int. Cl.: A61K 31/135

(54) **The use of sertraline to treat cancer patients**

(30) Priority: 17.01.1995 US 373148
(71) Applicant: PFIZER INC., New York, N.Y. 10017 (US)
(72) Inventor: Etienne, Pierre E., Old Lyme, Connecticut 06371 (US); Saxton, Craig, Lyme, Connecticut 06371 (US)
(74) Representative: Moore, James William, Dr.

(57) **Abstract**

The use of sertraline, or a pharmaceutically acceptable salt thereof, for the manufacture of a medicament for the treatment of cancer in a human being.

## Description

This invention relates to a method of treating human cancer patients comprising administering to such patients the compound (1S-cis)-4-(3,4-dichlorophenyl)-1,2,3,4-tetrahydro-N-methyl-1-naphthalenamine, hereinafter referred to by its generic name "sertraline", or a pharmaceutically acceptable salt thereof.

Sertraline, which has the empiral formula C₁₇H₁₇NCl₂ and the structural formula 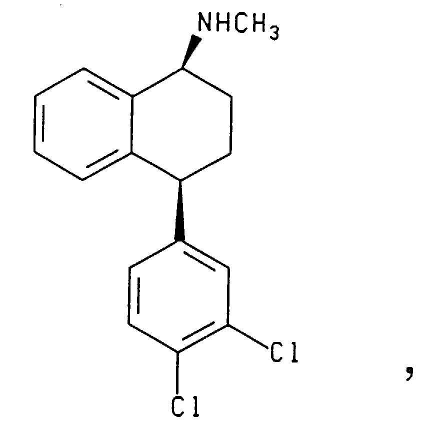 is a known antidepressant and anorectic agent. United States Patent 4,536,518, which issued on August 20, 1985, discloses sertraline and related compounds of the formula 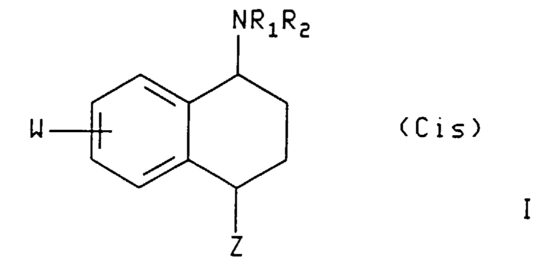 wherein Z is 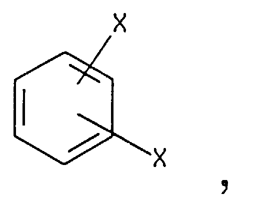 and R₁, R₂, W, X and Y are as defined therein, and states that such compounds exhibit antidepressant and anorectic activity in vivo in mammals.

United States Patent 5,130,338, which issued on July 14, 1992, refers to the use of sertraline to treat chemical dependencies, including dependencies on alcohol, tobacco and cocaine.

United States Patent 4,962,128, which issued on October 9, 1990, refers to the use of sertraline to treat disorders such as panic disorder, obsessive-compulsive disorder, generalized anxiety disorder, phobias, post traumatic stress disorder and avoidant personality disorder.

United States Patent 4,940,731, which issued on July 10, 1990, refers to the use of sertraline to treat premature ejaculation.

Examples of pharmaceutically acceptable salts of sertraline that can be used to treat cancer patients in accordance with the present invention are the acid addition salts of various mineral and organic acids such as hydrochloric, hydrobromic, hydroiodide, sulfuric, phosphoric, acetic, lactic, maleic, fumaric, citric, tartaric, succinic, and gluconic. Such salts may exist in one or more distinct crystalline forms or polymorphs, as well as in an amorphous state. Several crystalline polymorphs of the hydrochloride salt of sertraline are described in United States Patent 5,248,699, which issued on September 28, 1993.

Sertraline, its pharmaceutically acceptable salts and the different crystalline polymorphs of sertraline hydrochloride may be prepared as described in United States Patent 4,536,518, and particularly, in Example 2 of that patent, and in United States Patent 5,248,699.

All the foregoing U.S. patents are incorporated herein by reference in their entireties.

This invention relates to a method of treating human cancer patients comprising administering to such patients from about 12.5 mg/day to about 500 mg/day sertraline or a pharmaceutically acceptable salt thereof.

"Treating", as used herein, refers to either reducing the risk of mortality, improving the quality of life or retarding the progression of the cancer.

Patients that can be treated with sertraline according to the method of this invention include, for example, patients that have been diagnosed as having lung cancer, bone cancer, pancreatic cancer, skin cancer, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostrate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

The present inventors believe that sertraline will be useful in reducing the risk of mortality in human cancer patients. "Reducing the risk of mortality", as used herein, means effecting a clinically significant increase in the survival time of a cancer patient relative to the predicted survival time for such patient. The predicted survival time for a given patient will depend on the type of cancer and the stage (i.e., the progression) of the disease. Predicted survival times for different types of cancer, as generally recognized by those skilled in the art of medicine, may be obtained using the Kaplan-Meier Method for Estimating a Survival Distribution, as described by Richard Simon, "Design and Conduct of Clinical Trials", chapter 19 in "Cancer - Principals and Practice of Oncology", volume 1, 4th edition, edited by DeVita et al., 1993, J.B. Uppincott Co., Philadelphia, or using another method or standard that is recognized by those skilled in the art. It is believed that sertraline will increase the chances of survival in human cancer patients regardless of whether they are suffering from depression, anxiety or a high level of stress.

The present inventors also believe that sertraline will be useful in retarding the progression of cancer in human cancer patients. "Retarding the progression of cancer", as used herein, refers to retarding or slowing the rate of proliferation of cancer cells. It is believed that sertraline will retard the progression of cancer in human cancer patients regardless of whether they are suffering from depression, anxiety or a high level of stress.

The present inventors also believe that sertraline will be useful in improving the quality of life of cancer patients. Quality of life can be determined according to any quality of life scale or standard that is recognized by those skilled in the art. Examples are the Katz Activities of Daily Living Scale (Katz et al., Int. J. Health Serv., 6, 493-507 (1976)) and the Sickness Impact Profile (Bergner et al., Med. Care, 14, 57-67 (1976)). The use of these and other parameters to evaluate the quality of life of patients in extremity sarcoma clinical trials is described by Sugarbaker et al., Surgery,91, 17-23 (1982). (The three foregoing literature references are incorporated herein by reference in their entireties). Sertraline is expected to be useful in improving the quality of life of cancer patients regardless of whether they are suffering from depression, anxiety or a high degree of stress.

One embodiment of this invention relates to a method of treating a cancer patient who is not suffering from depression, anxiety or a high level of stress, comprising administering to such patient sertraline, or a pharmaceutically acceptable salt of sertraline, in an amount from about 12.5 mg/day to about 500 mg/day, preferably from about 25 mg/day to about 200 mg/day.

Sertraline, or a pharmaceutically acceptable salt thereof, when used to reduce the risk of mortality or to retard the progress of cancer in a human cancer patient may be administered either orally or parenterally. It is generally administered in dosages ranging from about 12.5 to about 500 mg per day, preferably from about 25 to about 200 mg per day, in single or divided doses, although variations will necessarily occur depending upon the condition of the subject being treated and the particular route of administration chosen. It may be administered either alone or in combination with pharmaceutically acceptable carriers by either of the above routes, and such administration can be carried out in both single and multiple dosages. More particularly, sertraline, or a pharmaceutically acceptable salt thereof, may be administered in a wide variety of different dosage forms, i.e., it may be combined with various pharmaceutically acceptable inert carriers in the form of tablets, capsules, lozenges, troches, hand candies, powders, sprays, aqueous suspensions, injectable solutions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, sertraline, or a pharmaceutically acceptable salt thereof, when used to treat a cancer patient, is present in such dosage forms at concentration levels ranging from about 0.5% to about 90% by weight of the total composition, i.e., in amounts that are sufficient to provide the desired unit dosage.

For purposes of oral administration, tablets containing various excipients such as sodium citrate, calcium carbonate and calcium phosphate may be employed along with various disintegrants such as starch, preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred fillers would also include lactose or milk sugar as well as high molecular weight polyethylene glycols. When aqueous suspensions and/or elixirs are desired for oral administration, the sertraline, or pharmaceutically acceptable salt thereof, may be combined with various sweetening or flavoring agents, coloring matter or dyes and, if so desired, emulsifying and/or suspending agents, together with diluents such as water, ethanol, propylene glycol, glycerin and various like combinations thereof.

For purposes of parenteral administration, solutions of sertraline, or a pharmaceutically acceptable salt thereof, in sesame or peanut oil or in aqueous propylene glycol or N,N-dimethylformamide may be employed, as well as sterile aqueous solutions of the water-soluble, non-toxic mineral and organic acid addition salts previously enumerated. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art.

A typical dry solid pharmaceutical composition is prepared by blending the following materials together in the proportions by weight specified below:

Cis-(1S)-N-methyl-4-(3,4-dichlorophenyl)-1,2,3,4-tetrahydro-1-naphthalenamine hydrochloride: 50
Sodium citrate: 25
Alginic acid: 10
Polyvinylpyrrolidone: 10
Magnesium stearate: 5
After the dried composition is thoroughly blended, tablets are punched from the resulting mixture, each tablet being of such size that it contains 100 mg of sertraline hydrochloride. Other tablets are also prepared in a similar fashion containing 5, 10, 25, and 50 mg of sertraline hydrochloride respectively, by using the appropriate amount of the naphthalenamine salt in each case.

Another typical dry solid pharmaceutical composition is prepared by combining the following materials together in the proportions by weight indicated below:
Cis-(1S)-N-methyl-4-(3,4-dichlorophenyl)-1,2,3,4-tetrahydro-1-naphthalenamine hydrochloride: 50
Calcium carbonate: 20
Polyethylene glycol, average molecular weight, 4000: 30
The dried solid mixture so prepared is then thoroughly agitated so as to obtain a powdered product that is completely uniform in every respect. Soft elastic and hard-filled gelatin capsules containing this pharmaceutical composition are then prepared, employing a sufficient quantity of material in each instance so as to provide each capsule with 50 mg of the active ingredient.

## Claims

1. The use of sertraline, or a pharmaceutically acceptable salt thereof, for the manufacture of a medicament for the treatment of cancer in a human being.

2. The use according to claim 1 wherein the medicament is administered in an amount containing from 12.5 mg/day to 500 mg/day of sertraline or a pharmaceutically acceptable salt thereof.

3. The use according to claim 2 wherein the medicament is administered in an amount containing from 25 mg/day to 200 mg/day of sertraline or a pharmaceutically acceptable salt thereof.

4. The use according to any one of claims 1 to 3 wherein the cancer is lung cancer.

5. The use according to any one of claims 1 to 3 wherein the cancer is pancreatic cancer.

6. The use according to any one of claims 1 to 3 wherein the cancer is breast cancer.

7. The use according to any one of claims 1 to 3 wherein the cancer is bone cancer.

8. The use according to any one of claims 1 to 3 wherein the cancer is skin cancer.

9. The use according to any one of claims 1 to 3 wherein the cancer is Hodgkin's disease or leukaemia.

10. The use according to any one of claims 1 to 3 wherein the cancer is stomach cancer, colon cancer, prostrate cancer or bladder cancer.
